# EUROPEAN PATENT SPECIFICATION

(11) **EP 0 617 633 B1**
(45) Date of publication and mention of the grant of the patent: **01.12.1999**
(21) Application number: 93922153.7
(22) Date of filing: 03.09.1993
(51) Int. Cl.: A61M 29/00

(54) **DETACHABLE EMBOLIC COIL ASSEMBLY**
ANORDNUNG EINER LÖSBAREN EMBOLIESPIRALFEDER
ENSEMBLE SPIRALE EMBOLIQUE DETACHABLE

(30) Priority: 22.09.1992 US 949095; 13.11.1992 US 975376
(43) Date of publication of application: 05.10.1994
(60) Divisional application: 99102433.2
(73) Proprietor: TARGET THERAPEUTICS, Fremont, CA 94538 (US)
(72) Inventor: PALERMO, Thomas, J., Palo Alto, CA 94303 (US); PHAM, Phong, San Jose, CA 95116 (US)
(74) Representative: Price, Nigel John King
(86) International application number: US9308346
(87) International publication number: WO9406502

(56) References cited:
- WO-A-91/17789
- WO-A-92/21400
- WO-A-93/11825
- US-A- 4 512 338
- US-A- 4 830 003
- US-A- 5 037 427
- US-A- 5 109 867
- US-A- 5 122 136
- US-A- 5 217 484
- US-A- 5 261 916

## Description

This invention is an embolic coil and a device for delivering embolic coils to a selected site within the vasculature of the human body via use of a catheter. In particular, the device involves an embolic coil having a radially enlarged member attached to one end, which coil is released by forcing the radially enlarged member axially through a distendible aperture situated on the distal end of a pusher assembly.

The endovascular treatment of a variety of vascular maladies throughout the body is an increasingly more important form of therapy. Catheters have been used to place various treatment materials, devices, and drugs within arteries and veins in the human body. Examples of these devices and their use in such treatments are shown in U.S. Patent No. 5,234,437 ("Detachable Pusher-Vasoocclusive Coil Assembly with Threaded Coupling"). This document shows methods and devices for delivery of coils or wires within the human body to sites such as aneurysms, to occlude those sites. Coils such as are discussed in this document (as well as in U.S. Patent No. 4,994,069), may be of a regular or helical configuration or may assume a random convoluted configuration at the site. The coils normally are made of a radiopaque, biocompatible metal such as platinum, gold, tungsten, or alloys of these and other metals. In treating an aneurysm it is common to place a number of coils within the aneurysm. The coils occlude the site by posing a physical barrier to blood flow and by promoting thrombus formation at the site.

Coils have typically been placed at the desired site within the vasculature using a catheter and a pusher. The site is first accessed by the catheter. In treating peripheral or neural conditions requiring occlusion, the sites are accessed with flexible, small diameter catheters such as those shown in U.S. Patent Nos. 4,739,768 and 4,813,934 may be used. The catheter may be guided to the site through the use of guidewires (see U.S. Patent No. 4,884,579) or by the use flow-directed means such as balloons placed at the distal end of the catheter. Use of guidewires involves the placement of relatively long, torqueable proximal wire sections within the catheter attached to more flexible distal end wire sections designed to be advanced across sharp bends at vessel junctions. The guidewire is visible using x-ray and allows a catheter to be placed in vessels taking extremely tortuous paths, even when those vessel are surrounded by soft tissue such as the brain.

Once the chosen site has been reached, the catheter lumen is cleared by removing the guidewire (if a guidewire has been used), and the coil is placed into the proximal open end of the catheter and advanced through the catheter with a pusher. Pushers are wires having a distal end that is adapted to engage and push the coil through the catheter lumen as the pusher is advanced through the catheter. When the coil reaches the distal end of the catheter, it is discharged from the catheter by the pusher into the vascular site. This technique of discharging the coil from the distal end of the catheter has a number of undesirable limitations. First, because of the plunging action of the pusher and the coil, the positioning of the coil at the site cannot be controlled to a fine degree of accuracy. Second, once the coil has left the catheter, it is difficult to reposition or retrieve the coil if such is desired.

Several techniques have been developed to enable more accurate placement of coils within a vessel. In one technique (U.S. Patent No. 5,122,136, issued June 16, 1992 and upon which the preamble of claim 1 is based) the coil is bonded via a metal-to-metal joint to the distal end of the pusher. The pusher and coil are made of dissimilar metals. The coil-carrying pusher is advanced through the catheter to the site and a low electrical current is passed through the pusher-coil assembly. The current causes the joint between the pusher and the coil to be severed via electrolysis. The pusher may then be retracted leaving the detached coil at an exact position within the vessel. In addition to enabling more accurate coil placement, the electric current may facilitate thrombus formation at the coil site. The only perceived disadvantage of this method is that the electrolytic release of the coil requires a period of time so that rapid detachment of the coil from the pusher does not occur.

Another technique for detaching an embolic coil is shown in U.S. Patent No. 5,261,916. In that document, a coil having an enlarged portion is mated with a pusher having a keyway adapted to receive the enlarged portion of the coil in an interlocking relationship. The junction between the pusher and the coil is covered by a coaxial member. The coaxial member is movable by sliding the member axially. As the coaxial member is moved away from the junction where the coil's member engages the keyway of the pusher, the coil disengages and the pusher may be removed.

Another device for placement of coils is shown in U.S. Patent No. 5,234,437. This device includes a coil having a helical portion at one end and a pusher which is threaded to the inside of the helical coil by the use of a threaded section on the outside of the pusher. The device operates to discharge the coil by engaging the proximal end of the coil with a sleeve while the pusher is unthreaded. Once the pusher is free, the sleeve may be used to push the coil out into the treatment area.

Another method of placing an embolic coil is shown in U.S. Patent No. 5,108,407. This patent shows the use of a device in which embolic coils are separated from the distal end of a catheter by the use of heat-releasable adhesive bonds. The coil adheres to the therapeutic device via a mounting connection. Laser energy is transferred through a fiber optic cable which terminates at the connector. The connector becomes warm and releases the adhesive bond between the connector and the coil.

US Patent No. 3,334,629, to Cohn, suggests the use of a pusher having a socket to push an occlusive device within the inferior vena cava. However, the device's rounded end is not used to retain the occlusive device within the end of the inserter.

WO-A-92/21400 and WO-A-93/11825 are both relevant as prior art against the present invention under Article 54(3) EPC at most. Both references disclose detachable pusher-vasoocclusive coil assemblies in which the proximal end of the vasoocclusive coil is provided with a ball of smaller diameter than the coil. This ball is interlocked, either in a keyway in or between a pair of radially expandable jaws provided at the distal end of a pusher. The pusher-vasoocclusive coil assembly is delivered via a catheter, the catheter preventing premature detachment. Only when the distal end of the pusher exits the distal end of the catheter does the ball disengage from the pusher to release the coil.

According to a first aspect of the present invention there is provided a detachable embolic coil comprising a coil having ends, an outer diameter and an enlarged member fixedly attached to one of said ends, the enlarged member having an outer diameter which is larger than the outer diameter of the coil.

According to a second aspect of the present invention there is provided a pusher-coil assembly for use in occluding a selected site within a vessel, the assembly comprising:
(a) a coil of the construction of the first aspect of the present invention, wherein the enlarged member is fixedly attached to the coil's proximal end;
(b) a pusher housing having a socket at its distal end, said socket having a throat aperture diameter smaller than the diameter of the enlarged member fixedly attached to the coil; and
(c) a plunger located within the pusher housing that is axially movable relative to the pusher housing and coil from a first position to a second position which pushes the coil and the enlarged member through the socket throat and thus uncouples the coil from the pusher housing.

Embodiments of coil and pusher-coil assembly in accordance with the present invention will now be described, by way of example only, with reference to the accompanying drawings described below.
Figure 1 is an enlarged, partial sectional view of one variation of the pusher-coil assembly showing the coil after uncoupling.
Figures 2 and 3 show variations of the tip of the socket on the pusher housing.
Figure 4 is an enlarged view showing the distal end of the pusher housing, the plunger, and the ball on the coil engaged.
Figure 5 is an enlarged view showing the distal end of the pusher housing, the plunger, and the ball on the coil not engaged.
Figures 6, 7, and 8 show the procedure for reloading the pusher housing with another embolic coil.

In the drawings, the following convention is used: the proximal end is to the left and the distal end is to the right.

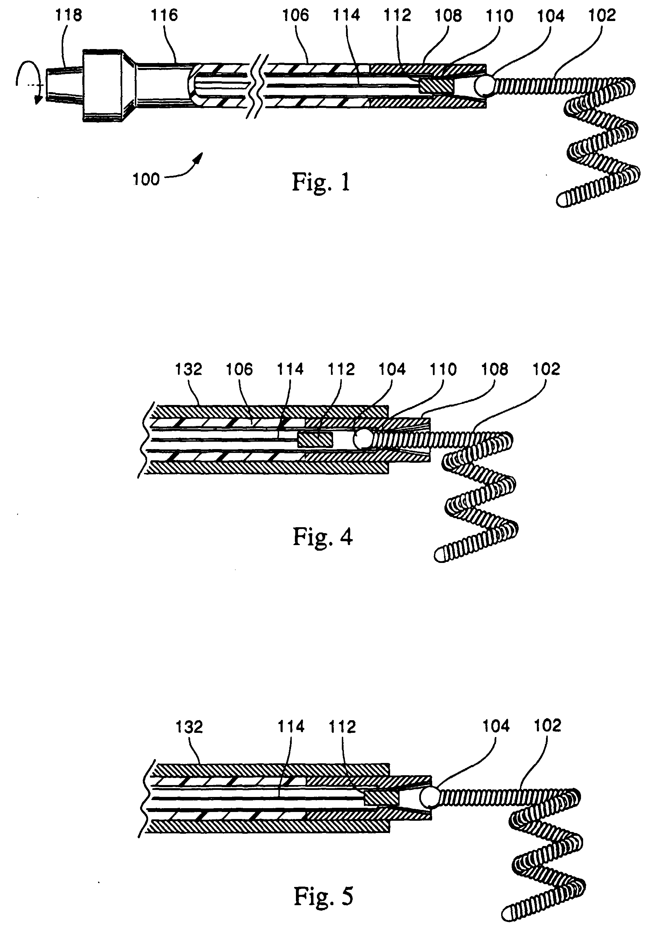

One variation of the pusher-coil assembly (100) is shown in Figure 1. The coil (102) is depicted to be helical in form, although it may be random or any other suitable form. The coil should be of a size sufficiently small that it may be advanced through a catheter that is appropriately sized for accessing the targeted vascular site. For instance, when accessing a brain aneurysm in a small vessel, an appropriately sized catheter is quite small and very flexible. The coil in such a situation must be small enough to fit through the catheter and out its distal end at the treatment site.

The coil is desirably made up of a radiopaque, physiologically compatible material. This material may be platinum, gold, tungsten, or alloys of these. A preferred material is a platinum or platinum/tungsten alloy. A number of polymers are also suitable as coil material either alone or in conjunction with metallic markers providing radiopacity, These materials are chosen so that the process of locating the coils within the vessel may be viewed using radiography. However, it is also contemplated that these coils may be made of various other biologically inert polymers or of carbon fiber.

The size of the coil and its constituent winding will depend upon the use to which the coil will be placed. For occluding peripheral or neural sites, the coils will typically be made of 0.05 to 0.15 mm diameter wire that is wound to have an inner diameter of 0.15 to 1.5 mm with a minimum pitch -- that is to say that the pitch is equal to the diameter of the wire used in the coil. The length of the coil will normally be in the range of 0.5 to 60 cm, preferably 0.5 to 40 cm.

If desired, the coil may be formed in such a way that the coil is essentially linear as it passes through the catheter and yet assume a randomly oriented relaxed condition after it is released from the distal end of the catheter. A discussion of this variation may be found in U.S. Patent No. 4,994,069.

Attached to coil (102) is a radially enlarged member, or ball (104). Ball (104) is firmly attached to coil (102) and should not separate during the installation treatment nor thereafter. The remainder of assembly (100) is made up of a pusher housing (106) which is a sheath or tube extending from the proximal end of the assembly (100) to the distal end terminated by a distendible aperture, a socket (108). Socket (108) includes a necked-down portion, a throat (110), which throat has a distendible aperture with a diameter smaller than that of ball (104). The ball (104) is pushed through throat (110) of socket (108) by a plunger head (112). Plunger head (112) easily fits within the aperture of throat (110) so to push ball (104) with its attached coil (102) out into the target site. The socket may have a constant inner diameter instead of the varying diameter shown in Figure 1. Plunger head (112) is pushed via a pusher wire (114). Pusher wire (114) may, as is shown in Figure 1, have a larger diameter at the proximal end of the assembly than at the distal end of the assembly near plunger head (112). In other variations, the diameter of pusher wire (114) may be constant throughout. The pusher wire (114) is desirably actuated by a screw-driven apparatus (116) and (118) in which as a knob (118) is rotated, the pusher wire (114) is advanced axially, distally down through the assembly (100) to push ball (104) out of the aperture (110) of socket (108).

The length of assembly (100) will be such as to be capable of being advanced entirely through the catheter to place coil (102) at the target site but yet with a sufficient portion of the proximal end of the assembly (100) protruding from the proximal end of the catheter to enable the plunger to be manipulated. For use in peripheral or neural surgeries, the pusher will normally be about 100-200 cm in length, more normally 130-180 cm in length. The diameter of the pusher housing is usually in the range of 0.25 to about 0.90 mm.

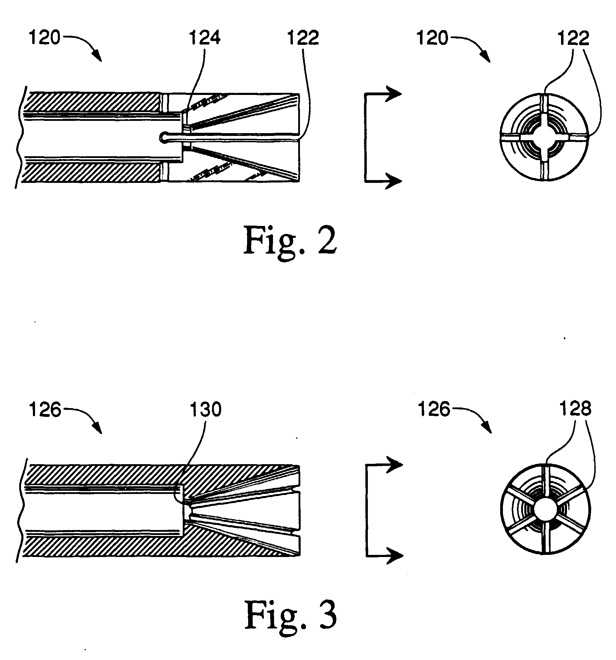

Two variations of the socket are shown in Figures 2 and 3. These variations are optional and are intended to lower the force needed to press ball (104) out through the throat of the socket aperture and yet hold the ball otherwise in a set position. In Figure 2, socket (120) incorporates a number of slots (122) which extend through the wall of the socket and terminate down near the resting place of the ball. This variation allows the ball to be firmly held inside of the socket throat (124) and yet be ejected easily using the plunger apparatus shown in Figure 1. Figure 3 similarly shows side cross-sectional views and end views of a socket which has grooves (128) cut from the distal end of the socket down into the aperture area (130). In each of Figures 2 and 3, the respective throat diameters (124) and (130) are each smaller than the diameter of the ball which is placed through them.

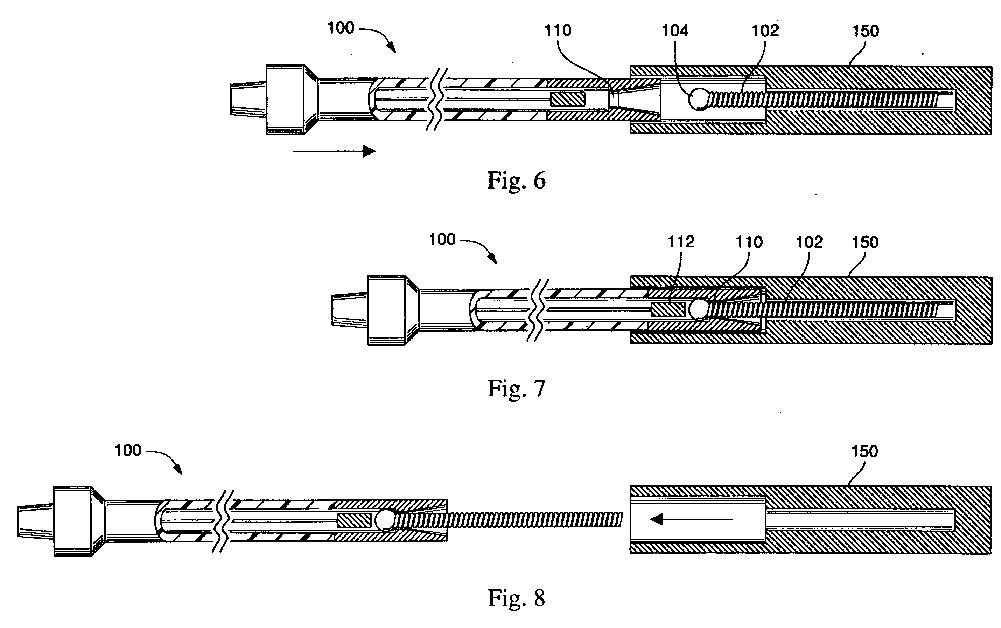

Assembly (100) is used to place one or more coils at the target site generally using the procedure as follows, As is shown in Figure 4, the coil (102) with its attached ball (104) are included into socket (108) with the ball pushed past socket throat (110). Catheter (132) is inserted and navigated through to the chosen vessel site. The assembly (100) is then included into the catheter lumen to the site to be occluded.

As indicated previously, conventional catheter insertion and navigational techniques involving guidewires or flow-directed devices may be used to access the site with a catheter. Once the distal end of the catheter is positioned at the site, often by locating its distal end through the use of radiopaque materials of construction and radiography, the catheter is cleared. For instance, if a guidewire has been used to position the catheter, it is withdrawn from the catheter and then the assembly (100) is advanced through the catheter. The assembly (100) is advanced past the distal end of the catheter (132) so that the coil is free of the catheter and with the coil positioned precisely at the desired treatment site. As is shown in Figure 5, plunger wire (114) is advanced to press the ball (104) and its attendant coil (102) into the target site. The entire catheter may then be removed or the assembly (100) may be withdrawn from the catheter lumen to provide for installation of other coils. If additional coils are to be placed at the target site, the procedure is repeated. After the desired number of coils have been placed at the site, the catheter is withdrawn from the vessel.

Figures 6, 7, and 8 show a method for reloading the assembly (100). Figure 6 shows a coil introducer (150) which includes coil (102) and a ball (104). The coil introducer (150) is cylindrical and adapted to hold a coil (102) and a ball (104) in such a fashion as to allow entry of assembly (100) to one end and allow engagement of throat (110) over ball (104). As is shown in Figure 7, the plunger head (112) is positioned out of the way as the ball is pressed through throat (110) into the position shown there. After the introduction of the ball (104) is complete, assembly (100) is withdrawn from coil introducer (150) as is shown in Figure 8, then placed in a catheter lumen and passed axially along to the target site as described above.

## Claims

1. A detachable embolic coil (102) comprising a coil having ends and an outer diameter, characterized by an enlarged member (104) fixedly attached to one of said ends, the enlarged member having an outer diameter which is larger than the outer diameter of the coil.

2. The coil of claim 1, wherein the enlarged member (104) is a ball.

3. The coil of claim 1 or claim 2, wherein the coil material is selected from platinum, gold, tungsten, or alloys of these.

4. The coil of claim 1 or claim 2, wherein the coil material is a polymer.

5. The coil of claim 1 or claim 2, wherein the coil material is carbon fibre.

6. The coil of any one of the preceding claims wherein the coil (102) is helical, random, or straight.

7. A pusher-coil assembly (100) for use in occluding a selected site within a vessel, the assembly comprising:
(a) a coil (102) of the construction claimed in any one of the preceding claims, wherein the enlarged member (104) is fixedly attached to the coil's proximal end;
(b) a pusher housing (106) having a socket (108) at its distal end, said socket having a throat aperture diameter smaller than the diameter of the enlarged member (104) fixedly attached to the coil (102); and
(c) a plunger (112) located within the pusher housing (106) that is axially movable relative to the pusher housing and coil (102) from a first position to a second position which pushes the coil and the enlarged member (104) through the socket throat (110) and thus uncouples the coil from the pusher housing.

8. The assembly of claim 7, wherein the distal socket end is slotted.

9. The assembly of claim 7, wherein the socket throat is grooved.

10. The assembly of any one of claims 7 to 9, additionally comprising a pusher wire (114).

11. The assembly of claim 10, additionally comprising means for advancing the pusher wire (114) and its plunger head (112).

## Patentansprüche

1. Lösbare Emboliespiralfeder (102), welche eine Spiralfeder mit Enden und einem Außendurchmesser umfaßt, gekennzeichnet durch ein vergrößertes Element (104), das fest an einem der Enden angebracht ist, wobei das vergrößerte Element einen Außendurchmesser aufweist, der größer als der Außendurchmesser der Spiralfeder ist.

2. Spiralfeder nach Anspruch 1, dadurch gekennzeichnet, daß das vergrößerte Element (104) eine Kugel ist.

3. Spiralfeder nach Anspruch 1 oder 2, dadurch gekennzeichnet, daß das Material der Spiralfeder aus Platin, Gold, Wolfram oder Legierungen derselben gewählt ist.

4. Spiralfeder nach Anspruch 1 oder 2, dadurch gekennzeichnet, daß das Material der Spiralfeder ein Polymer ist.

5. Spiralfeder nach Anspruch 1 oder 2, dadurch gekennzeichnet, daß das Material der Spiralfeder Graphitfaser ist.

6. Spiralfeder nach einem der vorstehenden Ansprüche, dadurch gekennzeichnet, daß die Spiralfeder (102) von schraubenförmiger, willkürlicher oder gerader Form ist.

7. Vorschieber-Spiralfederanordnung (100) zur Verwendung beim Verschluß eines gewählten Orts in einem Gefäß, wobei die Anordnung folgendes umfaßt:
(a) eine Spiralfeder (102) der in einem der vorstehenden Ansprüche beanspruchten Bauart, dadurch gekennzeichnet, daß das vergrößerte Element (104) fest an dem proximalen Ende der Spiralfeder angebracht ist;
(b) ein Vorschiebergehäuse (106) mit einem Flansch (108) an seinem distalen Ende, wobei der Flansch einen Durchlaßöffnungsdurchmesser aufweist, der kleiner als der Durchmesser des fest an der Spiralfeder (102) angebrachten vergrößerten Elements (104) ist, und
(c) einen in dem Vorschiebergehäuse (106) angeordneten Vorschieber (112), der relativ zum Vorschiebergehäuse und zur Spiralfeder (102) von einer ersten Position zu einer zweiten Position axial beweglich ist, was die Spiralfeder und das vergrößerte Element (104) durch den Flanschdurchlaß (110) schiebt und so die Spiralfeder von dem Vorschiebergehäuse löst.

8. Anordnung nach Anspruch 7, dadurch gekennzeichnet, daß das distale Flanschende einen Schlitz aufweist.

9. Anordnung nach Anspruch 7, dadurch gekennzeichnet, daß der Flanschdurchlaß eine Nut aufweist.

10. Anordnung nach einem der Ansprüche 7 bis 9, welche weiterhin einen Vorschieberdraht (114) aufweist.

11. Anordnung nach Anspruch 10, welche weiterhin Mittel zum Vorbewegen des Vorschieberdrahts (114) und dessen Vorschieberkopfs (112) umfaßt.

## Revendications

1. Serpentin embolique détachable (102) comprenant un serpentin ayant des extrémités et un diamètre extérieur, caractérisé par un élément agrandi (104) attaché de manière fixe à une desdites extrémités, l'élément agrandi ayant un diamètre extérieur qui est supérieur au diamètre extérieur du serpentin.

2. Serpentin selon la revendication 1, dans lequel l'élément agrandi (104) est une bille.

3. Serpentin selon la revendication 1 ou la revendication 2, dans lequel le matériau du serpentin est choisi parmi le platine, l'or, le tungstène, ou des alliages de ceux-ci.

4. Serpentin selon la revendication 1 ou la revendication 2, dans lequel le matériau du serpentin est un polymère.

5. Serpentin selon la revendication 1 ou la revendication 2, dans lequel le matériau du serpentin est de la fibre de carbone.

6. Serpentin selon l'une quelconque des revendications précédentes dans lequel le serpentin (102) est de forme en hélice, aléatoire, ou droite.

7. Ensemble poussoir-serpentin (100) destiné à être utilisé pour l'occlusion d'un site sélectionné dans un vaisseau, l'ensemble comprenant :
(a) un serpentin (102) ayant la construction selon l'une quelconque des revendications précédentes, dans lequel l'élément agrandi (100) est attaché de manière fixe à l'extrémité proximale du serpentin ;
(b) un logement de poussoir (106) ayant une douille (108) à son extrémité distale, ladite douille ayant une ouverture étranglée de diamètre inférieur au diamètre de l'élément agrandi (104) attaché de manière fixe au serpentin (102) ; et
(c) un plongeur (112) disposé à l'intérieur du logement de poussoir (106) qui est mobile axialement par rapport au logement de poussoir et au serpentin (102) depuis une première position vers une seconde position qui pousse le serpentin et l'élément agrandi (104) à travers l'étranglement de la douille (110) et ainsi désaccouple le serpentin du logement de poussoir.

8. Ensemble selon la revendication 7, dans lequel l'extrémité de douille distale est fendue.

9. Ensemble selon la revendication 7, dans lequel l'étranglement de douille est rainuré.

10. Ensemble selon l'une quelconque des revendications 7 à 9, comprenant en outre un fil poussoir (114).

11. Ensemble selon la revendication 10, comprenant en outre des moyens pour faire avancer le fil poussoir (114) et sa tête de plongeur (112).
